(19) Europäisches Patentamt
European Patent Office
Office européen des brevets

(11) Veröffentlichungsnummer: **0 282 448 A2**

# (12) EUROPÄISCHE PATENTANMELDUNG

(21) Anmeldenummer: **88810124.3**

(22) Anmeldetag: **29.02.88**

(51) Int. Cl.[4]: **C 07 D 231/06**
D 06 L 3/12, C 07 C 147/06

(30) Priorität: **05.03.87 CH 812/87**

(43) Veröffentlichungstag der Anmeldung:
**14.09.88 Patentblatt 88/37**

(84) Benannte Vertragsstaaten:
**BE CH DE FR IT LI NL**

(71) Anmelder: **CIBA-GEIGY AG**
**Klybeckstrasse 141**
**CH-4002 Basel (CH)**

(72) Erfinder: **Meyer, Hans Rudolf, Dr.**
**Bollwerkstrasse 102**
**CH-4102 Binningen (CH)**

(54) **Pyrazolinverbindungen.**

(57) Neue Pyrazolinverbindungen der Formel

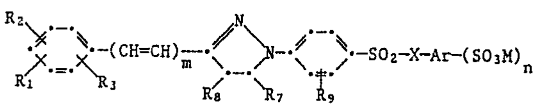

(1)

worin
X = 1,4-Phenylen, >C=CH₂, >CH-CH₂OH oder, falls n = 1 ist oder $R_7$ oder $R_8$ einen $SO_3M$-Rest aufweist, auch eine Methylen- , $-CH_2CH(OH)-CH_2O-$ oder eine unverzweigte $C_{1-4}$-Alkylenoxy-Gruppe, eine direkte Bindung oder Sauerstoff,
$R_1$, $R_2$, $R_3$ = unabhängig voneinander Wasserstoff, Chlor oder $C_{1-4}$-Alkyl,
$R_7$ = $C_{1-4}$-Alkyl, Chlorphenyl, Wasserstoff, Phenyl oder $-C_6H_4-SO_3M$
$R_8$ = H, $C_{1-4}$-Alkyl, $-CH_2SO_3M$, Carboxy, $C_{2-5}$-Carbalkoxy, Carbamoyl oder Carboxymethyl
$R_9$ = H, Chlor oder $C_{1-4}$-Alkyl
Ar = einen gegebenenfalls nicht chromophor substituierten Phenylrest oder falls X eine direkte Bindung ist auch einen Naphthalinrest,
M = Wasserstoff oder ein Aequivalent eines nichtchromophoren Kations und
m und n die Zahl Null oder 1 bedeuten,
die als optische Aufheller für synthetische und natürliche Fasern, besonders für Polyamid, verwendet werden können.
Verfahren zur Herstellung dieser Verbindungen, deren Verwendung als optische Aufheller und die Verbindungen enthaltende Mittel zum optischen Aufhellen von Fasern und Lacken aus Acetylcellulose, Wolle und insbesondere Polyamid, sowie neue Hydrazine als Zwischenprodukt bei der Herstellung der Verbindungen.

EP 0 282 448 A2

**Beschreibung**

Pyrazolinverbindungen

Gegenstand der Erfindung sind Pyrazolinverbindungen der Formel

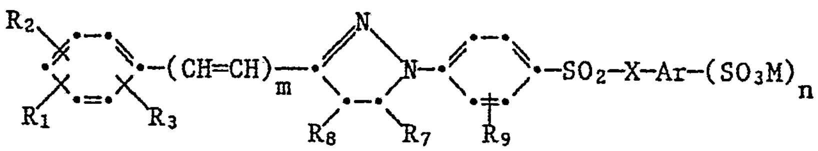

(1)

worin

X = 1,4-Phenylen, $>C=CH_2$, $>CH-CH_2OH$ oder, falls n = 1 ist oder $R_7$ oder $R_8$ einen $SO_3M$-Rest aufweist, auch eine Methylen-, $-CH_2\overset{OH}{C}H-CH_2O-$ oder eine unverzweigte $C_{1-4}$-Alkylenoxy-Gruppe, eine direkte Bindung oder Sauerstoff,
$R_1$, $R_2$, $R_3$ = unabhängig voneinander Wasserstoff, Chlor oder $C_{1-4}$-Alkyl,
$R_7$ = $C_{1-4}$-Alkyl, Chlorphenyl und besonders Wasserstoff, Phenyl oder $-C_6H_4-SO_3M$
$R_8$ = Carboxy, $C_{2-5}$-Carbalkoxy, Carbamoyl, Carboxymethyl und besonders H, $C_{1-4}$-Alkyl, $-CH_2SO_3M$
$R_9$ = $C_{1-4}$-Alkyl, insbesondere Wasserstoff, Methyl oder Chlor
Ar = einen gegebenenfalls nicht chromophor substituierten Phenylrest oder falls X eine direkte Bindung ist auch einen Naphthalinrest,
M = Wasserstoff oder ein Aequivalent eines nichtchromophoren Kations und
m und n die Zahl Null oder 1 bedeuten,
die als optische Aufheller für synthetische und natürliche Fasern, besonders für Polyamid, verwendet werden können.

Unter Alkyl sind sowohl geradkettige, wie verzweigte Kohlenstoffreste zu verstehen. Die Alkylenoxy-gruppe ist mit einem endständigen C-Atom an die Sulfonylgruppe gebunden. Als nichtchromophore Substituenten seien beispielsweise genannt: Phenylalkyl, Fluor, Alkoxy, alkyl, Benzyloxy, Phenyl, Cycloalkyl, Alkenyl, Alkenyloxy, Alkylsulfonyl, Phenylsulfonyl, Benzylsulfonyl, Carboxy, Carbalkoxy, Acyloxy, Carbamoyl, Sulfamoyl, Cyano, Acylamino und besonders $C_{1-4}$-Alkyl, $C_{1-4}$-Alkoxy, Phenoxy und Chlor. Die Verbindungen der Formel (1) weisen vorzugsweise nicht mehr als eine Sulfogruppe auf d.h. z.B. falls $R_7$ eine Sulfophenylgruppe oder $R_8$ eine Sulfomethylgruppe bedeutet, so ist n = 0. Zwei benachbarte Alkylgruppen können auch einen Tetralyl- oder Indanyl-ring bilden.

M in Formel (1) steht beispielsweise für Erdalkalimetall, wie Magnesium oder Calcium, vorzugsweise jedoch für Wasserstoff, Alkalimetall wie Lithium, Natrium, Kalium und gegebenenfalls substituiertes Ammonium, wie Ammonium, Mono-, Di- oder Triäthanolammonium, Mono-, Di- oder Triisopropanol-ammonium oder Tri- oder Tetraethyl-ammonium.

Bevorzugt sind die Verbindungen, worin n = 1 bedeutet. Die Sulfogruppen aufweisenden haben gegenüber der sulfogruppen-freien Vertretern den Vorteil der Wasserlöslichkeit. Besonders hohe Weisseffekte zeigen Verbindungen, worin X eine direkte Bindung bedeutet.

Bevorzugte Verbindungen der Formel (1) sind solche der Formel

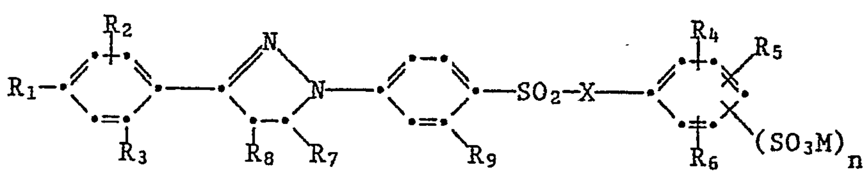

(2)

worin

X = 1,4-Phenylen, $>C=CH_2$, $>CH-CH_2OH$, oder falls n = 1 ist und/oder $R_8$ den Rest $-SO_3M$ aufweist, auch eine geradkettige $C_{1-4}$-Alkylenoxygruppe, Sauerstoff oder vorzugsweise eine direkte Bindung
$R_1$ bis $R_3$ = H, Cl, Methyl
$R_4$ und $R_5$ = H, Cl, $C_{1-4}$-Alkyl, $C_{1-4}$-Alkoxy, Phenoxy oder zusammen die Ergänzung zu einem Tetralin- oder Indanring,
$R_6$ = H oder Methyl
$R_7$ = H, Phenyl,
$R_8$ = H, oder $C_{1-4}$-Alkyl, $-CH_2SO_3M$
$R_9$ = H oder Cl
und n und M wie bei Formel (1) angegeben, bedeuten.

Die Erfindung bezieht sich besonders auf Pyrazolinverbindungen der Formel

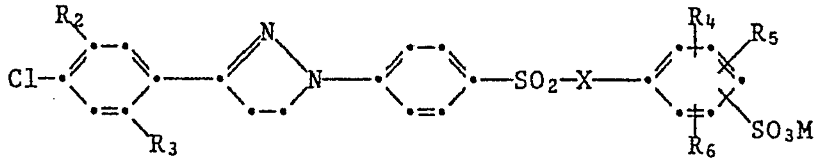

(3)

worin
X die Methylengruppe, eine geradkettige $C_{1-4}$-Alkylenoxygruppe oder vorzugsweise eine direkte Bindung
$R_2$ und $R_3$ = Wasserstoff, Chlor oder Methyl,
$R_4$ und $R_5$ = Wasserstoff, Chlor, $C_{1-4}$-Alkyl, Methoxy oder zusammen die Ergänzung zu einem Tetralinring,
$R_6$ und M die in Formel (2) angegebene Bedeutung haben
und vor allem auf Pyrazolinverbindung der Formel

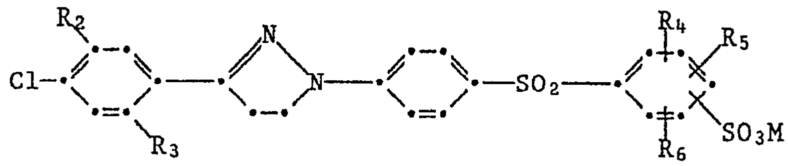

(4)

worin
$R_2$ = Wasserstoff oder besonders Chlor
$R_3$ = Wasserstoff oder besonders Methyl
$R_4$ = Wasserstoff oder $C_{1-4}$-Alkyl
$R_5$ und $R_6$ = Wasserstoff oder Methyl und
M die in Formel (2) angegebene Bedeutung haben.

Die Erfindung umfasst auch Mischungen der hier beanspruchten Verbindungen. Durch Kombination von geeigneten rotstichigen mit grünstichigen Vertretern lassen sich beliebige Nuancen von Weisseffekten erzeugen.

1-Phenyl-pyrazolinverbindungen mit Sulfoalkylsulfonylsubstituenten in p-Stellung zum Phenylrest sind bereits aus den DE 1 419 329, 1 670 988, 1 719 355, 2 011 552, 2 142 564, 2 516 053, 2 524 927 und 2 641 814 sowie Sulfoarylsulfonamide aus der DE 2 403 308 bekannt. Die erfindungsgemässen Pyrazolinverbindungen zeichnen sich gegenüber diesen Typen besonders durch bessere Weisseffekte aus.

Die Herstellung der erfindungsgemässen Pyrazolinverbindungen erfolgt durch ein Verfahren der Umsetzung einer Verbindung der Formel

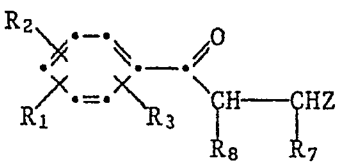

(5) bzw.

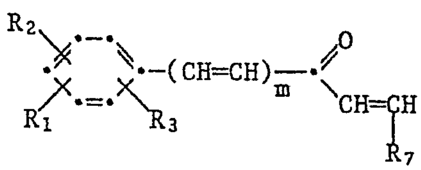

(6)

worin Z Abgangsgruppen wie Halogen, eine Di-$C_{1-4}$-Alkylaminogruppe oder einen Morpholino-, Pyrrolidino- oder Piperidinorest bedeuten, in an sich bekannter Weise, mit Hydrazinen der Formel

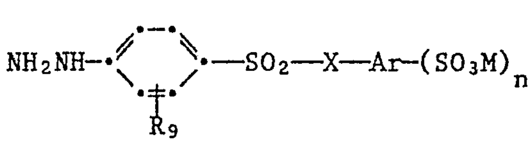

(7)

Die Umsetzung erfolgt vorteilhaft in wässrigem, wässrig-organischem oder organischem Medium, bei Temperaturen von 50-120° C. Das Mischungsverhältnis wird dabei vorzugsweise so gehalten, dass die Edukte noch genügend gelöst werden und die Produkte ausfallen. Geeignete organische Lösungsmittel sind z.B. Alkohole, wie Methanol, Ethanol, Isopropanol, n-Propanol, sek.-Butanol, n-Butanol, Ethylenglykolmonomethylether, sowie Dimethylformamid oder Essigsäure.

Nach einem weiteren Verfahren führt man Pyrazolinsulfosäuren der Formel

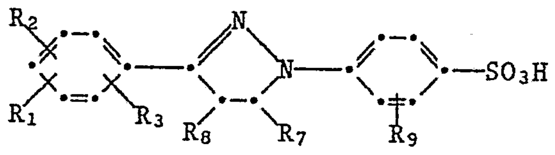

(8)

oder deren Salze in bekannter Weise via Sulfochlorid (DAS 1 695 103) in die Sulfinsäuren oder deren Salze über und kondensiert diese mit Verbindungen der Formel

Z-X-Ar-$(SO_3M)_n$ (9)

worin Z Halogen bedeutet, in geeigneten Fällen (für n = Null) unter Nachsulfierung. Dieses Verfahren ist besonders geeignet für Pyrazoline der Formel 1, worin m und n Null und X eine gegebenenfalls substituierte Methylengruppe bedeutet.

Die Hydrazine der Formel 7, worin n = Null ist, sind teilweise bekannten bzw. können nach bekanntem Verfahren hergestellt werden (DE 1 285 886, J. pr. Chem., 132 (1931) S. 34-36, Chem. Abs. 53 (1959) 8122i).

Die Hydrazine der Formel 7, worin n = 1 bedeutet, sind neu und stellen einen weiteren Erfindungsgegenstand dar. Man erhält sie, indem man Aniline der Formel

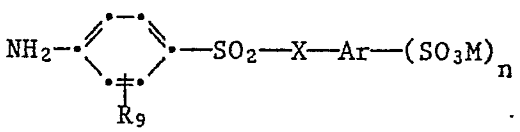

(10)

in an sich bekannter Weise diazotiert und die Diazoniumverbindungen reduziert. Die Reduktion erfolgt vorteilhaft mit Sulfit in neutralem bis leicht alkalischem Medium, wonach das Reaktionsprodukt mit Säure nachbehandelt wird.

Die Aniline der Formel 10, worin X eine direkte Bindung bedeutet, erhält man z.B. aus p-Nitrochlorbenzolen und Benzolsulfinsäuren gemäss EP 101 664. Die Sulfierung der als Zwischenprodukte entstehenden p-Nitrophenyl-sulfone erfolgt z.B. mit Oleum, $SO_3$, Schwefelsäure oder Chlorsulfonsäure, gegebenenfalls unter anschliessender Verseifung des gebildeten Sulfochlorids. Bevorzugt ist die Umsetzung mit Oleum bei 0 bis 100°C.

Nach einem anderen Verfahren, wobei X = $CH_2$ ist [Helv. Chim. Acta 66 (1983) 1046-1053], wird p-Acetaminobenzolsulfinsäure mit Benzylhalogeniden umgesetzt, die erhaltenen Sulfone verseift und gewünschtenfalls, wie vorangehend beschrieben, sulfiert oder vorteilhaft zuerst sulfiert und dann verseift (GB 2 006 252). Gemäss einem 3. Verfahren wird p-Acetaminobenzolsulfochlorid in Gegenwart von Friedel-Crafts-Katalysatoren mit geeigneten Aromaten zu den entsprechenden Sulfonen kondensiert [Gazz. chim. ital. 79 (1949) 621-9;], welche sodann wie vorangehend beschrieben, weiter umgesetzt werden. Umgekehrt gelangt man ebenfalls zu Sulfonen, indem man Acetanilid mit substituierten Benzolsulfochloriden in Gegenwart von Aluminiumchlorid kondensiert (CH 278 939).

Nach einer weiteren, an sich bekannten Methode erhält man Hydrazine der Formel 7 worin n = 1 bedeutet, entsprechend den unsulfierten Vertretern, durch Umsetzung der Sulfone der Formel

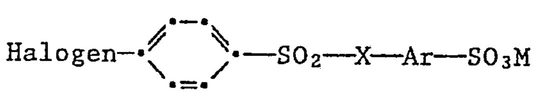

(11)

worin Halogen Cl, Br oder I bedeutet, mit Hydrazin. Dabei verwendet man z.B. überschüssiges Hydrazinhydrat bei 90°C-125°C; gegebenenfalls z.B. bei Schwerlöslichkeit von (11), unter Zusatz eines geeigneten Lösungsmittels wie Aethylenglykolmonomethyläther.

Die Sulfone der Formel (11) erhält man z.B. durch Friedel-Crafts-reaktion von Chlorbenzol mit Arylsulfonylchloriden (DRP 701954; J. Applied. Chem. USSR 50 (1977) 1532-5) oder umgekehrt von p-Chlorphenylsulfonylchlorid mit geeigneten Aromaten (DOS 2'038'167; Chem. Ber. 109 (1976) 2315-26). Die Sulfierung der erhaltenen p-Chlorphenylsulfone erfolgt alsdann wie bei den p-Nitrophenylsulfonen z.B. mit Oleum (siehe Beispiel 2, Methode d).

Die erfindungsgemässen Verbindungen eignen sich zum Aufhellen verschiedener Substrate. Die in Wasser unlöslichen Vertreter zeigen gute Eigenschaften beim Aufhellen von Fasern und Lacken aus Polyamid, Acetylcellulose und Wolle. Besonders geeignet sind die anionischen Verbindungen zum Aufhellen von Polyamid und Wolle bei der textilen Applikation. Auch durch Behandlen im Waschbad, wird die Polyamidfaser aufgehellt.

Es können auch Mischungen dieser optischen Aufheller eingesetzt werden, wie sie z.B. schon bei der Synthese anfallen.

Beispiel 1:

Zu einer Lösung von 9,0 g des Hydrazins der Formel

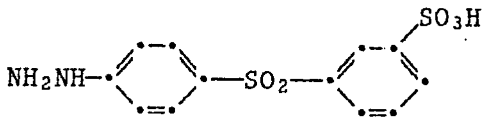 (101)

(Gehalt 91 %) in 25 ml n-Propanol und 25 ml Wasser tropft man bei 70°C eine warme Lösung von 7,9 g 3,3',4'-Trichlor-6'-methyl-propiophenon in 25 ml n-Propanol. Durch allmähliche Zugabe von 30%iger Natronlauge hält man den pH bei 2-3. Man verrührt die Lösung noch 1 Stunde bei 70°C und dann über Nacht bei Rückflusstemperatur. Alsdann neutralisiert man auf pH 8 und lässt abkühlen. Das ausge fallene Produkt wird abgesaugt mit 2%iger Kochsalzlösung und mit n-Propanol gewaschen und im Vakuum bei 100°C getrocknet. Man erhält 6,0 g der Verbindung der Formel

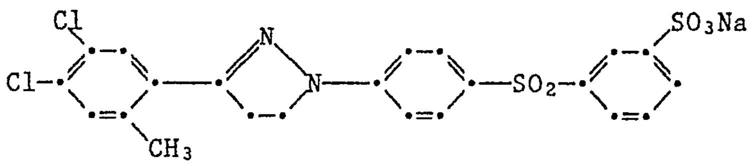 (102)

die aus 70%igem wässrigem n-Propanol umkristallisiert wird.

Das als Ausgangsprodukt verwendete Hydrazin der Formel (101) kann wie folgt hergestellt werden: 235 g der Verbindung der Formel

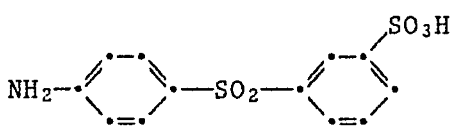 (103)

werden in 940 ml heissem Wasser gelöst. Man fügt unter heftigem Rühren 110,8 g konzentrierte Salzsäure hinzu, kühlt ab und tropft bei 5-10°C eine Lösung von 52,5 g Natriumnitrit in 75 ml Wasser hinzu. Man lässt eine Stunde bei derselben Temperatur nachrühren, korrigiert nötigenfalls mit Sulfaminsäure bzw. Natriumnitrit auf Kaliumiodid-Stärke-Umschlag und tropft die Diazoniumlösung unter Rühren bei Raumtemperatur im Verlauf von etwa 20 Min. in 585,3 g einer wässrigen 40%igen Natriumbisulfitlösung, die zuvor mit 30%iger Natronlauge (ca. 150 ml) auf pH 7 gebracht worden ist. Nach Ablauf 1 Stunde erwärmt man die erhaltene Lösung auf 60°C und tropft vorsichtig 443,4 g konzentrierte Salzsäure hinzu, so dass die Schwefeldioxidentwicklung unter Kontrolle gehalten werden kann. Nach 1-stündigem Nachrühren bei Rückflusstemperatur wird auf 5°C abgekühlt, das ausgefallene Produkt abgenutscht, mit Wasser und Methanol gewaschen und im Vakuum bei 80°C getrocknet. Man erhält 241,5 g eines farblosen Pulvers.

Beispiel 2:

Gemäss Beispiel 1 erhält man die Hydrazine der allgemeinen Formel (104) als freie Sulfosäuren und daraus durch Kondensation mit den entsprechenden β-Chlorpropiophenonen die Pyrazoline der allgemeinen Formel (105) in Form ihrer Natriumsalze Tab. 1). Die Sulfogruppen aufweisenden Hydrazine der Formel (104) können oft besser durch umgekehrte Diazotierung, d.h. durch Zugabe einer wässrigen Lösung des Alkalisalzes des zugrunde liegenden Sulfsäure und Natriumnitrit zu wässriger Salzsäure hergestellt werden. Als Lösungsmittel für die Umsetzung der Hydrazine verwendet man eine Mischung n-Propanol-Wasser von 20:3. Falls nicht speziell erwähnt, bedeuten die Substituenten $R_8$ und $R_9$ Wasserstoff.

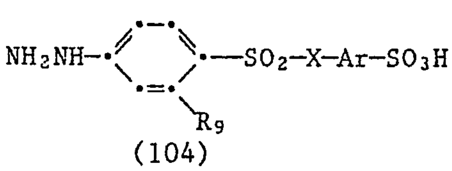

(104)

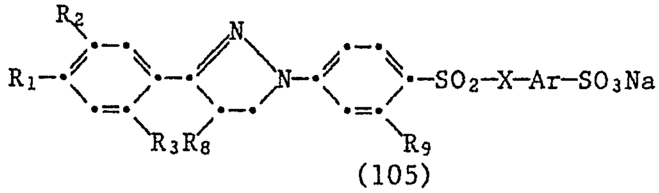

(105)

Tabelle I

| (105) $R_1$ $R_2$ $R_3$ | (104) bzw. (105) $-X-Ar-SO_3H(Na)$ | (104) umkrist. aus | Herst. methode | (105) Formel |
|---|---|---|---|---|
| Cl H H | 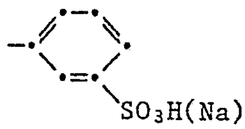 | $H_2O$ | a) | (106) |
| Cl Cl H | " | | | (107) |
| Cl H H | 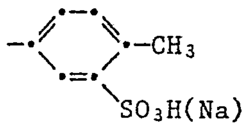 | n-Propanol $-H_2O$ 1:1 | a) | (108) |
| Cl Cl H | " | | | (109) |
| Cl H Cl | " | | | (110) |
| Cl Cl $CH_3$ | " | | | (111) |
| H H H | " | | | (112) |
| Cl H H | 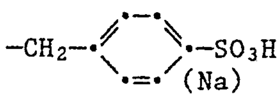 | - | b) c) | (113) |
| Cl Cl H | " | | | (114) |
| Cl Cl $CH_3$ | " | | | (115) |
| Cl H H | 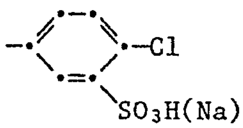 | n-Propanol $-H_2O$ 1:1 | a) | (116) |
| Cl Cl H | " | | | (117) |
| Cl Cl $CH_3$ | " | | | (118) |
| Cl H H | 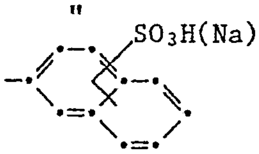 | - | a) | (119) |
| Cl Cl $CH_3$ | 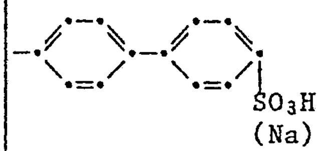 | n-Propanol $-H_2O$ 1:1 | c) | (120) |
| Cl H H | 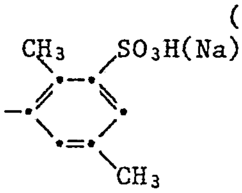 | n-Propanol $-H_2O$ 1:1 | b) d) | (121) |
| Cl Cl H | " | | | (122) |
| Cl Cl $CH_3$ | " | | | (123) |

Fortsetzung Tabelle I

| (105) $R_1$ $R_2$ $R_3$ | (104) bzw. (105) $-X-Ar-SO_3H(Na)$ | (104) umkrist. aus | Herst. methode | (105) Formel |
|---|---|---|---|---|
| Cl H H | 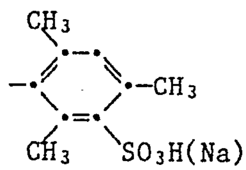 | n-Propanol $-H_2O$ 1:1 | b) | (124) |
| Cl Cl H | " | | | (125) |
| Cl Cl $CH_3$ | " | | | (126) |
| Cl H H | 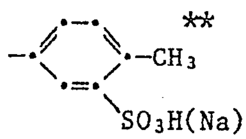 | | a) | (127) |
| Cl H H | 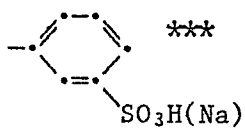 | - | a) | (128) |
| Cl H H | 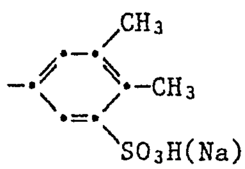 | n-Propanol $-H_2O$ 1:1 | a) | (129) |
| Cl Cl H | " | | | (130) |
| Cl Cl $CH_3$ | " | | | (131) |
| Cl H H | 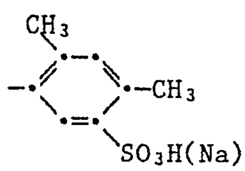 | n-Propanol $-H_2O$ 1:1 | a) | (132) |
| Cl Cl H | " | | | (133) |
| Cl Cl $CH_3$ | " | | | (134) |
| Cl H H | 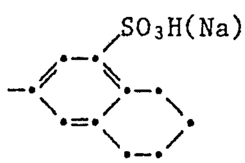 | - | a) | (135) |
| Cl Cl H | " | | | (136) |
| Cl Cl $CH_3$ | " | | | (137) |

Fortsetzung Tabelle I

| (105) $R_1$ $R_2$ $R_3$ | (104) bzw. (105) $-X-Ar-SO_3H(Na)$ | (104) umkrist. aus | Herst. methode | (105) Formel |
|---|---|---|---|---|
| Cl H H | 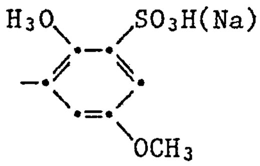 | MC*-$H_2O$ 1:1 | c) | (138) |
| Cl Cl H | " | | | (139) |
| Cl Cl $CH_3$ | " | | | (140) |
| Cl H H | 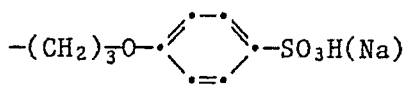 | MC*-$H_2O$ 1:1 | d) | (141) |
| Cl Cl H | " | | | (142) |
| Cl Cl $CH_3$ | " | | | (143) |
| Cl Cl $CH_3$ | 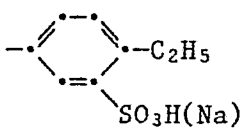 | n-Propanol -$H_2O$ 1:1 | d) | (144) |
| Cl Cl $CH_3$ | 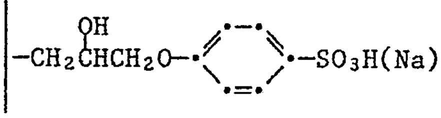 | $H_2O$ | d) | (145) |

*MC = Aethylenglykolmonomethyläther
**$R_8$ = $CH_3$
***$R_9$ = Cl

Die zur Herstellung der Hydrazine von Tab. I der allgemeinen Formel (104) benötigen Sulfosäuren der Formel (10) erfolgte nach 3 verschiedenen Methoden

Method a:
Sulfierung der Nitroverbindungen der Formel

(150)

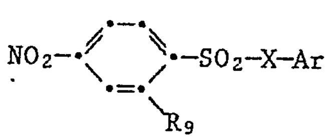

mit 25 %-igem Oleum bei 20-60° C (Temperatur je nach Reaktionsfähigkeit) und anschliessende Reduktion mit Eisen in verdünnter wässriger Essigsäure nach Bechamp gemäss EP 101 664 oder 102 325.

Methode b:
Sulfierung der Aniline der Formel

(151)

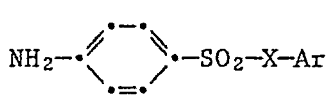

mit Oleum, wie vorangehend beschrieben.

Methode c:
Sulfierung der Acetylaniline der Formel

(152) 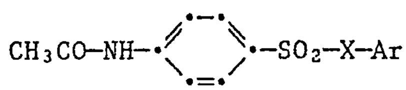

wie unter b. Nach Austragen der Reaktionsmasse auf Eiswasser wird 1 bis 2 Stunden auf Rückflusstemperatur erhitzt, wobei Verseifung entritt. Ausnahmen: Zur Sulfierung der Verbindung der Formel

(153) 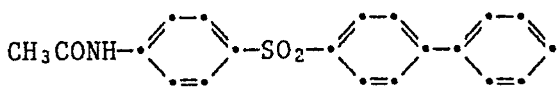 Smp. 240°C (Acetonitril)

Smp. 240°C (Acetonitril)
genügt die Verwendung von konz. Schwefelsäure anstelle von Oleum (1 Stunde Bei 60°C). Die Verbindung wird erhalten durch Friedel-Crafts Reaktion von Biphenyl mit 4-Acetaminobenzolsulfochlorid in Nitrobenzol in Gegenwart von Aluminiumchlorid bei 70-100°C.

Die zur Herstellung der Hydrazine von Tab. I der allgemeinen Formel (104) erforderlichen Sulfosäuren der Formel (11) erfolgte nach

Methode d:
Chlorverbindungen der Formel

(154) 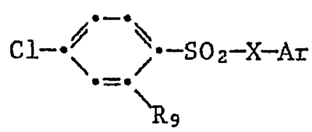

werden mit Oleum oder Schwefelsäure wie vorangehend beschrieben sulfiert.

Die Sulfierung der Verbindung der Formel

(155) 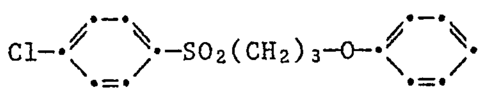

Smp. 74-5°C, erfolgt mit konz. Schwefelsäure (1 Stunde bei Raumtemperatur). Nach austragen auf Eis und Aussalzen mit Kochsalz erhält man die Verbindung der Formel

(156) 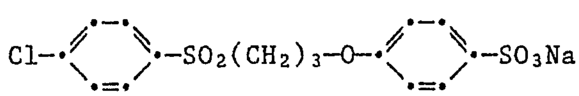

umkrist. aus n-Propanol-Wasser 4:1.

Zur Herstellung der Verbindung

(157) 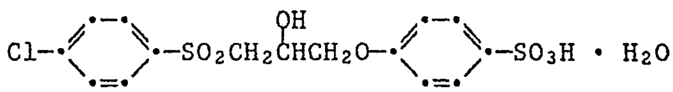

verrührt man 13,1 g der Verbindung der Formel

(158) 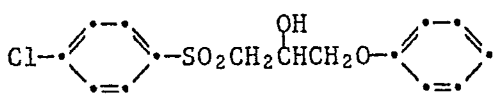

in 50 ml konz. Schwefelsäure 2 Stunden bei Raumtemperatur, trägt die Lösung auf Eis aus, sodass ein

Gesamtvolumen von 190 ml entsteht und erhitzt die Lösung 1 Stunde bei 100° C. Nach dem Abkühlen auf 0° C wird das ausgefallene Produkt abgenutscht, wiederholt mit Aceton gewaschen und im Hochvakuum getrocknet. Ausbeute 8,4 g.

Das Ausgangsprodukt der Formel (158) erhält man durch Eintropfen von Phenylglycidäther (20 % Ueberschuss) in eine Lösung von Natrium-4-chlorbenzolsulfinat in wässrigem Alkohol bei 70° C bei pH 7-8 unter fortlaufender Neutralisation der frei gesetzten Natronlauge mit konz. Salzsäure. Nach beendeter Reaktion wird die erhaltene Lösung im Vakuum vollständig eingedampft, der Rückstand in Methylenchlorid-Wasser verrührt, die beiden Phasen im Scheidetrichter getrennt, die Methylenchlorid-Phase getrocknet und eingedampft und der Rückstand aus Aether oder Isopropanol kristallisiert, Smp. 77-8° C.

Das zur Herstellung der Verbindungen der Formeln (135) bis (137) benötigte Sulfon der Formel

(159) 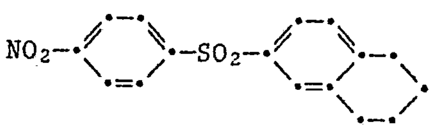

erhält man wie folgt:

Zu 114,4 g einer 40 %-igen Natriumbisulfitlösung, die mit 320 ml Wasser verdünnt und mit 30 %-iger Natronlauge auf pH 7-8 gestellt worden ist, trägt man unter heftigem Rühren bei 70° C 93,7 g 1,2,3,4-Tetrahydronaphthalin-6-sulfochlorid portionenweise ein. Durch gleichzeitiges Eintropfen von Natronlauge hält man den pH bei 7-8. Man lässt bei gleichbleibender Temperatur nachrühren bis keine weitere Natronlauge mehr verbraucht wird (Gesamtverbrauch ca 120 ml NaOH 30 %) und erwärmt noch 1 Std. bei 80° C. Die Lösung wird klärfiltriert, abgekühlt und unter weiterem Kühlen mit ca 50 ml konz. Salzsäure angesäuert. Das ausgefallene, farblose Produkt wird abgesaugt, mit 120 ml Wasser in 3 Portionen gewaschen und im Hochvakuum über Calciumchlorid bis zur Gewichtskonstanz getrocknet. Man erhält 78,5 g 1,2,3,4-Tetrahydronaphthalin-6-sulfinsäure (Smp. 82-5°).

In eine Suspension von 78,5 g dieser Sulfinsäure in 150 ml Dimethylsulfoxid trägt man unter heftigem Rühren vorsichtig (Schäumen) 21,2 g Soda ein. Zur erhaltenen Lösung fügt man 63,0 g 1-Chlor-4-nitrobenzol hinzu und verrührt die Mischung 4 Stunden bei 100° C. Man verdünnt noch in der Wärme mit 150 ml Aethanol und 300 ml Wasser und lässt abkühlen. Das ausgefallene Produkt wird abgesaugt und wiederholt mit Wasser und Methanol gewaschen. Man erhält 98,8 g der Verbindung der Formel (159), Smp. 135-6° (aus Isopropanol).

Beispiel 3:

Entsprechend Beispiel 2 erhält man die unsulfierten Hydrazine der allgemeinen Formel (201) und daraus die Pyrazoline der allgemeinen Formel (202) (Tab. 2). Die Herstellung der Hydrazine erfolgte nach Methode e) oder f).

Methode e):

Diazotierung und Reduktion der entsprechenden Aniline.

Methode f):

Umsetzung der entsprechenden p-Chlorphenylsulfone mit Hydrazinhydrat in Gegenwart von Methylcellosolve bei Rückflusstemperatur.

Tabelle 2

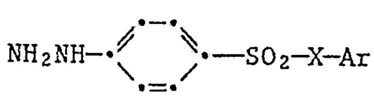

(201)

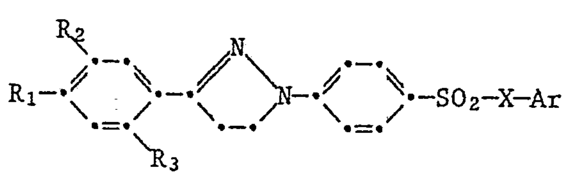

(202)

Tabelle 2

| (202) $R_1$ $R_2$ $R_3$ | (201) bzw. (202) -X-Ar | (201) Herst.-methode | (201) Smp. | (202) Smp. (°C) | (202) Formel |
|---|---|---|---|---|---|
| Cl H H | 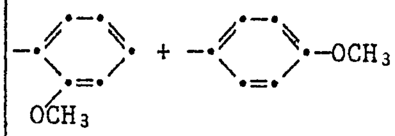 | e) | | 198-208° | (203) |
| Cl Cl H | 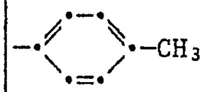 | e) | 151° | 208° | (204) |
| Cl Cl $CH_3$ | " | | | 250° | (205) |
| Cl H H | 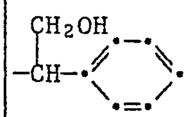 | e) | 177°(Zers.) | 166° | (206) |
| Cl H H | 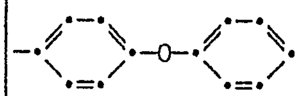 | f) | 191° | 205° | (207) |
| Cl H H | 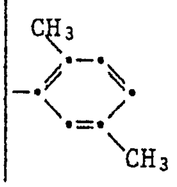 | f) | ca 120° | 228° | (208) |

Das zur Herstellung des Isomerengemisches der Formel (203) benötigte 4-(2'/4'-Methoxyphenylsulfonyl)-phenylhydrazin erhält man in üblicher Weise durch Diazotieren und Natriumsulfitreduktion der Isomerenmischung der Formel

(209) 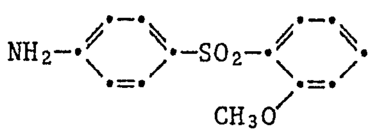 + 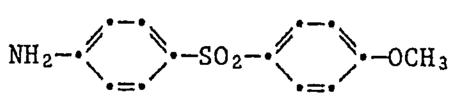

Smp. 185-188°C. Diese erhält man durch Friedel-Crafts-reaktion von 4-Acetylaminobenzolsulfochlorid mit überschüssigem Anisol in Gegenwart von Aluminiumchlorid bei 100-120°C und Verseifen des Reaktionsproduktes mit Salzsäure bei Rückflusstemperatur. Zur Isolierung wird die organische Phase abgetrennt, mit Wasser und Natronlauge gewaschen, überschüssiges Anisol und als Nebenprodukt gebildeter Sulfanilsäurephenylester durch Destillation im Hochvakuum entfernt und der Rückstand aus Essigester kristallisiert.

Das zur Herstellung der Verbindung der Formel (206) erforderliche Hydrazin erhält man wie folgt: Zu einer Lösung von 39,8 g p-Acetylaminobenzolsulfinsäure und 35,4 g Natriumacetat-trihydrat in 200 ml Aethanol tropft man bei Rückflusstemperatur unter rühren im Verlauf 1 Stunde 26,4 g Phenyläthylenoxid. Nach weiteren 5 Stunden Rückfluss lässt man abkühlen und fügt 300 ml Wasser hinzu. Das ausgefallene Produkt wird abgesaugt, wiederholt mit Wasser gewaschen und im Vakuum bei 100°C getrocknet. Man erhält 35,9 g der Verbindung der Formel

(210) 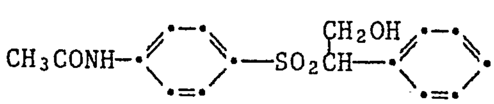

Smp. 193-4°C (umkrist. aus Acetonitril).

31,9 g der Verbindung der Formel (210) werden in 200 ml Wasser und 100 ml konz. Salzsäure 1 Stunde bei Rückflusstemperatur verrührt. Man versetzt die erhaltene Lösung mit Natronlauge bis pH ~12, wobei das Produkt ausfällt. Man erhält 26,5 g der Verbindung der Formel

(211) 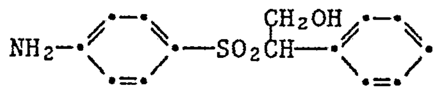

Smp. 252-3°C (umkrist. aus Aethylenglykolmonomethyläther).

16,4 g der Verbindung der Formel (211) werden in 60 ml Wasser und 57 ml konz. Salzsäure in der Hitze gelöst. Nach Abkühlen auf 0 bis 5°C tropft man unter Rühren im Verlauf von 15 Min. eine Lösung von 4,1 g Natriumnitrit in 10 ml Wasser hinzu. Man lässt noch 1 Stunde bei derselben Temperatur nachrühren und tropft die erhaltene Diazoniumsalzlösung unter Rühren zu 46,0 g einer wässrigen 450 %-igen Natriumbisulfitlösung, die zuvor mit 30 %-iger Natronlauge auf pH 7 gestellt worden ist. Durch gleichzeitige Zugabe weiterer Natronlauge hält man den pH bei 7. Gesamtverbrauch ca. 73 ml 30 %-ige Natronlauge. Nach Ablauf 1 Stunde erwärmt man die Lösung auf 60°C und tropft vorsichtig 34,9 g konz. Salzsäure hinzu (Schwefeldioxid-Entwicklung). Nach 3-stündigem Nachrühren bei Rückflusstemperaur wird die Lösung abgekühlt und mit Natronlauge auf pH 10 gestellt, wobei das gewünschte Hydrazin als freie Base ausfällt. Das Produkt wird abgesaugt, wiederholt mit Wasser gewaschen und im Vakuum bei 80°C getrocknet. Man erhält 16,1 g der Verbindung der Formel

(212) 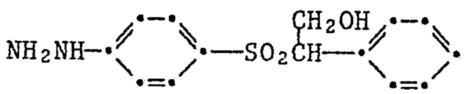

Smp. 177-178° (Zers.). Die Verbindung kann aus Aethanol umkristallisiert werden.

Beispiel 4:

Man trägt bei Raumtemperatur unter Rühren 11,0 g der Verbindung der Formel (206) in 30,0 g konz. Schwefelsäure ein und rührt 3 Stunden nach. Die Lösung wird in eine Mischung von Eis und Wasser gegossen, sodass ein Volumen von 200 ml resultiert. Nach verrühren mit 40 g Kochsalz wird das ausgefallene Produkt in der Kälte abgenutscht, in 100 ml Eiswasser verrührt und die Suspension durch Zutropfen von 30 %-iger Natronlauge auf pH 10-11 gebracht. Man lässt die Temperatur auf 80° ansteigen und hält den pH durch weitere Zugabe von Natronlauge konstant (Totalverbrauch 8,5 ml). Nach beendeter Reaktion wird abgekühlt, das ausgefallene Produkt abgenutscht, wiederholt mit Wasser und dann mit Methanol gewaschen. Man erhält 9,6 g der Verbindung der Formel

(213) 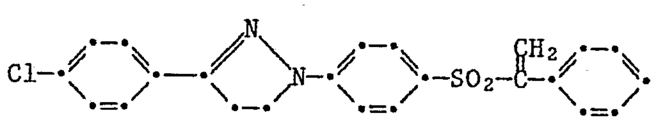

Smp. 183-4° (aus n-Butanol und Xylol).

Beispiel 5:

Man verrührt 7,3 g Benzal-4-chloracetophenon und 10,8 g des Hydrazins der Formel (101) (Gehalt 91 %) in 150 ml Aethanol, fügt 5 ml konz. Salzsäure hinzu und erhitzt die Mischung 18 Stunden bei Rückflusstemperatur. Man neutralisiert die erhaltene Lösung mit 30 %-iger Natronlauge auf pH 8 und lässt langsam abkühlen. Das ausgefallene Produkt wird abgesaugt, mit Aethanol gewaschen und im Vakuum bei 100°C getrocknet. Man erhält 16,6 g der Verbindung der Formel

(301) 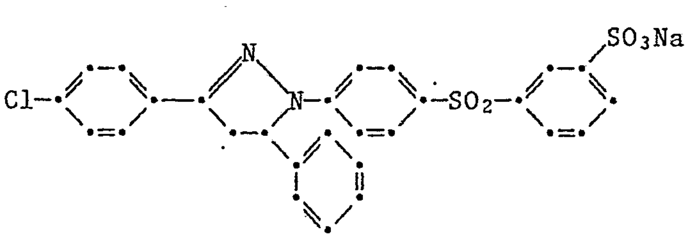

als annähernd farbloses Pulver. Die Reinigung erfolgt durch Umkristallisation aus einer Mischung von n-Propanol-Wasser 7:3 unter Zuhilfenahme von Aktivkohle.

Verwendet man in diesem Beispiel anstelle von Benzal-4-chloracetophenon die äquivalente Menge Dibenzalaceton, so erhält man die Verbindung der Formel

(302) 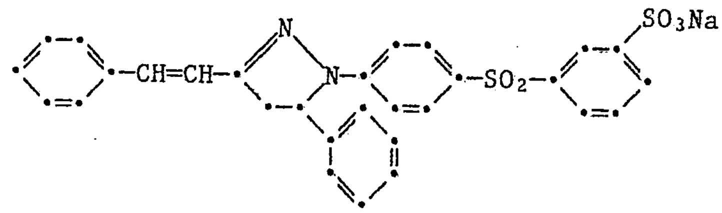

sie wird aus Methanol-Aethanol umkristallisiert.

Beispiel 6:

Kondensiert man das interne Salz der Mannich-Base 3-(4″-Morpholino)-2-sulfomethyl-3′,4′-dichlor-6′-methyl-propionphenon mit den entsprechenden Hydrazinen in Aethylenglykolmonomethyläther/Wasser in Gegenwart von Natriumacetat bei Rückflusstemperatur, so erhält man die Verbindungen der Formeln

(303) 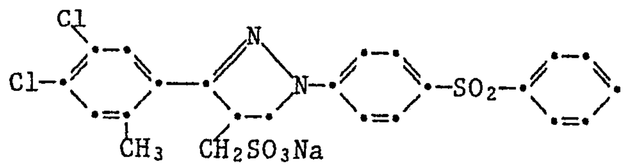

(304) 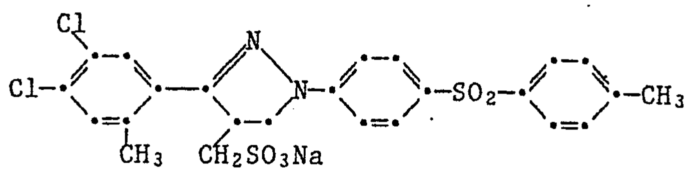

oder in analoger Weise die Verbindung der Formel

(305) 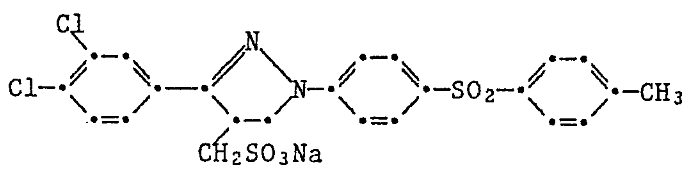

Beispiel 7:

Zu einer Lösung von 4,7 g p-Phenolsulfosäure Na-salz in 12,5 ml Aethanol und 5,25 ml 2 n Natronlauge fügt man portionenweise 3,55 g der Verbindung der Formel

(310) 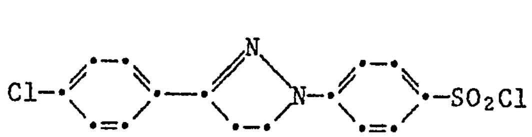

Man verrührt zunächst bei Raumtemperatur und alsdann bei Rückflusstemperatur unter gleichzeitigem Zutropfen von wenig weiterer Natronlauge bis ein konstanter pH von 8 erreicht ist. Nach dem Abkühlen wird das Produkt abgenutscht, mit Alkohol gewaschen und getrocknet. Zur Reinigung wird es bei Raumtemperatur in Aethylenglykolmonomethyläther aufgenommen, die Lösung durch Filtration von unlöslichem Material befreit und am Rotationsverdampfer im Vakuum vollständig eingedampft. Nach Auskochen des Rückstandes mit Alkohol und Absaugen bei Raumtemperatur erhält man die Verbindung der Formel

(311) 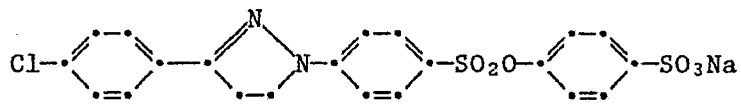

In analoger Weise erhält man die Verbindungen der Formeln

(312) 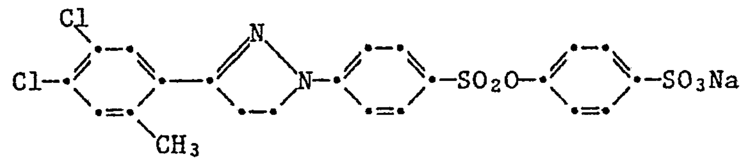 und

(313) 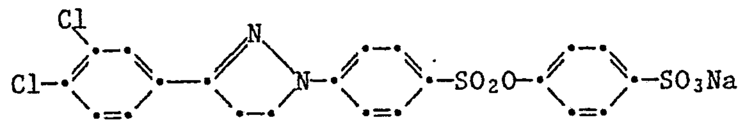

Beispiel 8:

Entsprechend den vorangehenden Beispielen können die folgenden Hydrazine der Formel (104) und daraus die Pyrazoline der Formel (105) hergestellt werden, wobei $R_8$ und $R_9$ Wasserstoff bedeuten: (Tab. 3)

Tabelle 3

| (105) $R_1$ | $R_2$ | $R_3$ | (104) bzw. (105) $-X-Ar-SO_3H(Na)$ | Formel (105) |
|---|---|---|---|---|
| Cl | H | H | 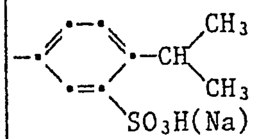 | (401) |
| Cl | Cl | H | " | (402) |
| Cl | Cl | $CH_3$ | " | (403) |
| Cl | H | H | 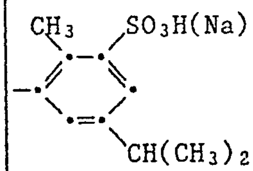 | (404) |
| Cl | Cl | H | " | (405) |
| Cl | Cl | $CH_3$ | " | (406) |
| Cl | H | H | 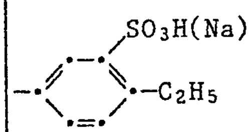 | (407) |
| Cl | Cl | H | " | (408) |
| Cl | H | H | 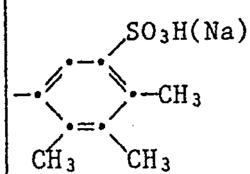 | (409) |
| Cl | Cl | H | " | (410) |
| Cl | Cl | $CH_3$ | " | (411) |
| Cl | H | H | 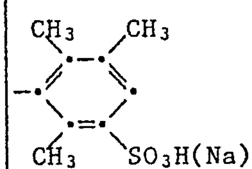 | (412) |
| Cl | Cl | H | " | (413) |
| Cl | Cl | $CH_3$ | " | (414) |
| Cl | H | H | 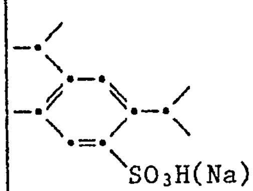 | (415) |
| Cl | Cl | H | " | (416) |
| Cl | Cl | $CH_3$ | " | (417) |

Fortsetzung Tabelle 3

| (105) $R_1$ | $R_2$ | $R_3$ | (104) bzw. (105) $-X-Ar-SO_3H(Na)$ | Formel (105) |
|---|---|---|---|---|
| Cl | H | H | 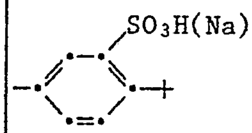 | (418) |
| Cl | Cl | H | " | (419) |
| Cl | Cl | $CH_3$ | " | (420) |
| Cl | H | H | 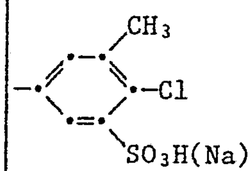 | (421) |
| Cl | Cl | H | " | (422) |
| Cl | Cl | $CH_3$ | " | (423) |
| Cl | H | H | 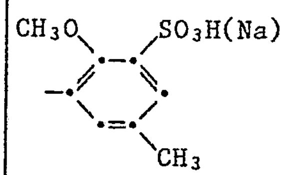 | (424) |
| Cl | Cl | H | " | (425) |
| Cl | Cl | $CH_3$ | " | (426) |
| Cl | H | H | 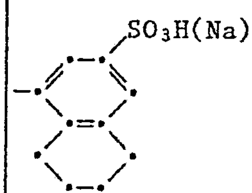 | (427) |
| Cl | Cl | $CH_3$ | " | (428) |
| Cl | H | H | 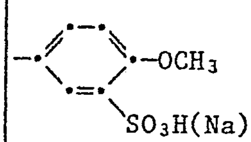 | (429) |
| Cl | Cl | H | " | (430) |
| Cl | Cl | $CH_3$ | " | (431) |
| Cl | H | H | 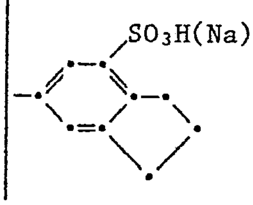 | (432) |

Fortsetzung Tabelle 3

| (105) $R_1$ | $R_2$ | $R_3$ | (104) bzw. (105) $-X-Ar-SO_3H(Na)$ | Formel (105) |
|---|---|---|---|---|
| Cl | Cl | H | " | (433) |
| Cl | Cl | $CH_3$ | " | (434) |
| Cl | H | H | 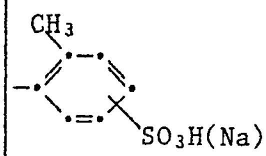 | (435) |
| Cl | Cl | H | " | (436) |
| Cl | Cl | $CH_3$ | " | (437) |
| Cl | H | H | 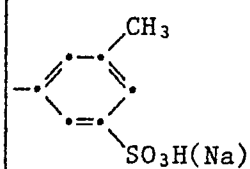 | (438) |
| Cl | Cl | H | " | (439) |
| Cl | Cl | $CH_3$ | " | (440) |
| Cl | H | H | 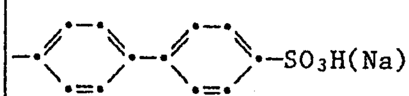 | (441) |
| Cl | H | H | 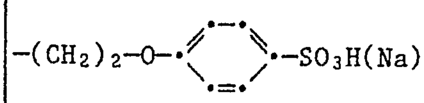 | (442) |
| Cl | Cl | H | " | (443) |
| Cl | Cl | $CH_3$ | " | (444) |
| Cl | Cl | $CH_3$ | 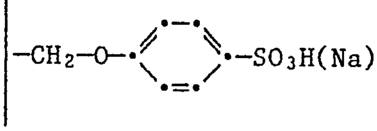 | (442) |

Beispiel 9:

Ein Polyamid-6,6-gewebe wird auf einem Färbeapparat bei einem Flottenverhältnis von 1:20 mit einem wässrigen Bad behandelt, das 0,05 % bezogen auf das Gewebegewicht einer Verbindung der Formel (102), (109), (111), (123), (126), (131), (134) oder einer 1:1-Mischung von (109) und (111), 3 g/l eines Anlagerungsproduktes von 1 Mol Stearylalkohol und 35 Mol Aethylenoxid, 3 g/l gepuffertes Natriumdithionit und 1 ml/l Essigsäure 80 % enthält. Die Applikation erfolgt gemäss folgendem Temperaturprogramm: 40-97° innerhalb 30 Minuten, 100° innerhalb 30 Minuten und 100-40° innerhalb von 15 Minuten. Anschliessend wird das Gewebe in enthartetem Wasser gespült und getrocknet. Es weist einen hohen Aufhelleffekt auf.

Beispiel 10:

Man foulardiert bei Raumtemperatur ein Polyamidgewebe (Nylon Webtricot Typ 6) mit einer wässrigen Flotte, die 0,5 g/l eines in Beispiel 9 genannten Aufhellers, sowie 1 g/l eines Anlagerungsproduktes von 1 Mol Stearylalkohol an 35 Mol Aethylenoxid, 1 g/l eines Anlagerungsproduktes von 1 Mol p-tert.Octylphenol an 8 Mol Aethylenoxid, 90 ml Aethanol 95% und 5 ml/l Essigsäure 80 % enthält. Die Flottenaufnahme beträgt

110 %. Anschliessend wird getrocknet und 40 Sekunden bei 190° thermofixiert. Das so behandelte Gewebe weist einen hohen Aufhelleffekt auf.

Beispiel 11:

Gebleichte Wollserge wird auf einem Färbeapparat bei einem Flottenverhältnis von 1:25 in einem wässrigen Bad behandelt, das 0,05 %, bezogen auf das Stoffgewicht, einer Verbindung der Formel (109), (111) oder (132), 3 g/l gepuffertes Natriumdithionit und 1 ml/l Essigsäure 80 % (Zugabe nach 45 Min.) enthält. Die Applikation erfolgt während 75 Min. bei 60°C. Nach dem Spülen in deionisiertem Wasser weist das Gewebe einen hohen Aufhelleffekt auf.

Beispiel 12:

Ein Gewebe aus Celluloseacetat wird im Flottenverhältnis 1:25 in ein wässriges Bad gebracht, das 0,1 % bezogen auf das Gewebegewicht der Verbindung der Formel (206) und 1 g/l äthoxylierten Fettalkohol enthält. Man bringt die Temperatur des Behandlungsbades auf 80°C und hält es während 30 Min. bei dieser Temperatur. Nach dem Spülen und Trocknen ist das Gewebe stark aufgehellt.

**Patentansprüche**

1. Pyrazolinverbindungen der Formel

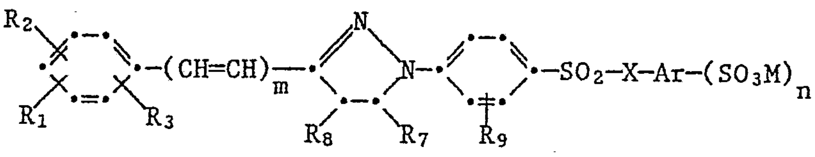

(1)

worin

X = 1,4-Phenylen, $>C=CH_2$, $>CH-CH_2OH$ oder, falls n = 1 ist oder $R_7$ oder $R_8$ einen $SO_3M$-Rest aufweist, auch eine Methylen- , $-CH_2CH(OH)-CH_2O-$ oder eine unverzweigte $C_{1-4}$-Alkylenoxy-Gruppe, eine direkte Bindung oder Sauerstoff,

$R_1$, $R_2$, $R_3$ = unabhängig voneinander Wasserstoff, Chlor oder $C_{1-4}$-Alkyl,

$R_7$ = $C_{1-4}$-Alkyl, Chlorphenyl, Wasserstoff, Phenyl oder $-C_6H_4-SO_3M$

$R_8$ = H, $C_{1-4}$-Alkyl, $-CH_2SO_3M$, Carboxy, $C_{2-5}$-Carbalkoxy, Carbamoyl oder Carboxymethyl

$R_9$ = H, Chlor oder $C_{1-4}$-Alkyl

Ar = einen gegebenenfalls nicht chromophor substituierten Phenylrest oder falls X eine direkte Bindung ist auch einen Naphthalinrest,

M = Wasserstoff oder ein Aequivalent eines nichtchromophoren Kations und

m und n die Zahl Null oder 1 bedeuten.

2. Pyrazolinverbindungen gemäss Anspruch 1, gekennzeichnet durch die Formel

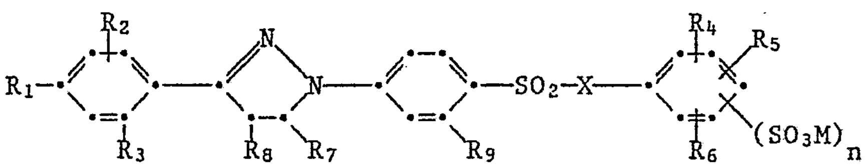

(2)

worin

X = 1,4-Phenylen, $>C=CH_2$, $>CH-CH_2OH$ , oder falls n = 1 ist und/oder $R_8$ den Rest $-SO_3M$ aufweist, auch eine geradkettige $C_{1-4}$-Alkylenoxygruppe, Sauerstoff oder eine direkte Bindung

$R_1$ bis $R_3$ = H, Cl, Methyl

$R_4$ und $R_5$ = H, Cl, $C_{1-4}$-Alkyl, $C_{1-4}$-Alkoxy, Phenoxy oder zusammen die Ergänzung zu einem Tetralin- oder Indanring,

$R_6$ = H oder Methyl

$R_7$ = H, Phenyl,

$R_8$ = H, $C_{1-4}$-Alkyl oder $-CH_2SO_3M$

$R_9$ = H oder Cl und

n und M die zur Formel 1 angegebene Bedeutung haben.

3. Pyrazolinverbindungen gemäss Anspruch 1, gekennzeichnet durch die Formel

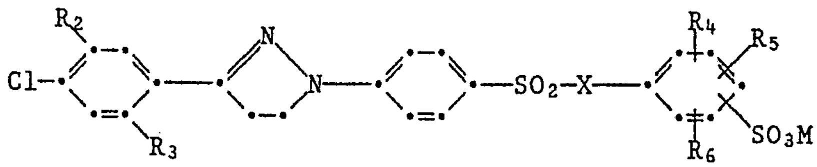

(3)

worin
X die Methylengruppe, eine geradkettige $C_{1-4}$-Alkylenoxygruppe oder eine direkte Bindung
$R_2$ und $R_3$ = Wasserstoff, Chlor oder Methyl,
$R_4$ und $R_5$ = Wasserstoff, Chlor, $C_{1-4}$-Alkyl, Methoxy oder zusammen die Ergänzung zu einem Tetralinring,
$R_6$ und M die in Formel (2) angegebene Bedeutung haben.

4. Pyrazolinverbindungen gemäss Anspruch 1, gekennzeichnet durch die Formel

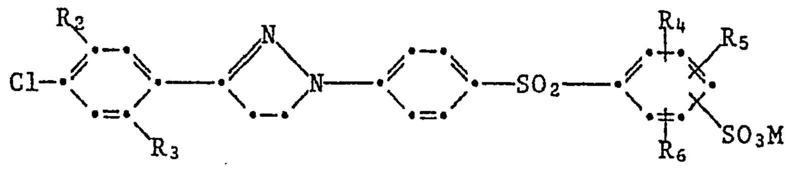

(4)

worin
$R_2$ = Wasserstoff oder Chlor
$R_3$ = Wasserstoff oder Methyl
$R_4$ = Wasserstoff oder $C_{1-4}$-Alkyl
$R_5$ und $R_6$ = Wasserstoff oder Methyl und
M die in Formel (2) angegebene Bedeutung haben.

5. Verfahren zur Herstellung von Pyrazolinverbindungen gemäss Anspruch 1, dadurch gekennzeichnet, dass eine der Verbindungen der Formeln

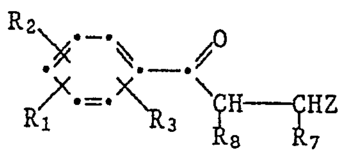

(5) oder

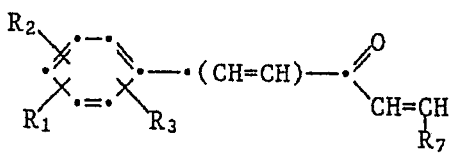

worin Z eine Abgangsgruppe wie Halogen, eine Di-$C_{1-4}$-Alkylaminogruppe oder einen Morpholino-, Pyrrolidino- oder Piperidinorest bedeuten, mit Hydrazinen der Formel

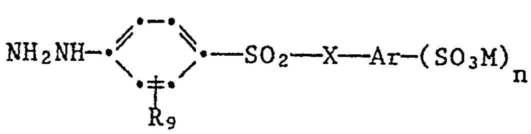

(7)

worin X, $R_1$ bis $R_9$, Ar, M und n die zur Formel 1 angegebenen Bedeutungen haben, gegebenenfalls unter Nachsulfierung, wenn n = Null, umgesetzt wird.

6. Verfahren zur Herstellung von Pyrazolinverbindungen gemäss Anspruch 1, in denen m = Null und $R_7$ und $R_8$ = H sind, dadurch gekennzeichnet, dass Pyrazolinsulfosäuren der Formel

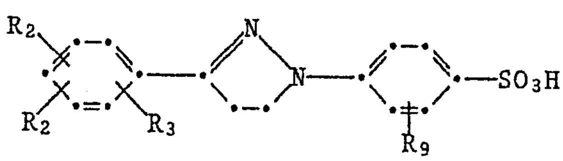

(8)

oder deren Salze über das Sulfochlorid zu Sulfinsäuren oder deren Salze umgesetzt und diese mit Verbindungen der Formel
$Z\text{-}X\text{-}Ar\text{-}(SO_3M)_n$ (9)

worin X, $R_1$ bis $R_9$, Ar, M und n die zur Formel 1 angegebenen Bedeutungen haben und Z Halogen bedeutet, kondensiert und gegebenenfalls, wenn n = Null ist, nachsulfiert werden.

7. Hydrazine der Formel

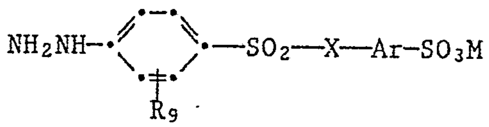

(7)

worin X, $R_9$, Ar und M die zur Formel 1 angegebenen Bedeutungen haben.

8. Verwendung von Pyrazolinverbindungen der Formel

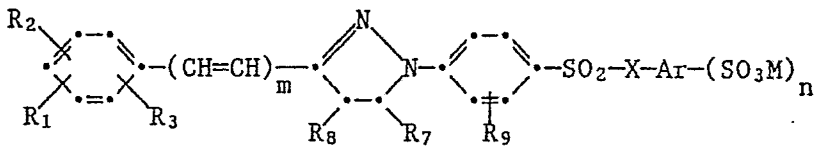

(1)

worin

X = 1,4-Phenylen, $>C=CH_2$, $>CH\text{-}CH_2OH$ oder, falls n = 1 ist oder $R_7$ oder $R_8$ einen $SO_3M$-Rest aufweist, auch eine Methylen- , $-CH_2CH(OH)-CH_2O-$ oder eine unverzweigte $C_{1-4}$-Alkylenoxy-Gruppe, eine direkte Bindung oder Sauerstoff,
$R_1$, $R_2$, $R_3$ = unabhängig voneinander Wasserstoff, Chlor oder $C_{1-4}$-Alkyl,
$R_7$ = $C_{1-4}$-Alkyl, Chlorphenyl, Wasserstoff, Phenyl oder $-C_6H_4-SO_3M$
$R_8$ = H, $C_{1-4}$-Alkyl, $-CH_2SO_3M$, Carboxy, $C_{2-5}$-Carbalkoxy, Carbamoyl oder Carboxymethyl
$R_9$ = H, Chlor oder $C_{1-4}$-Alkyl
Ar = einen gegebenenfalls nicht chromophor substituierten Phenylrest oder falls X eine direkte Bindung ist auch einen Naphthalinrest,
M = Wasserstoff oder ein Aequivalent eines nichtchromophoren Kations und
m und n die Zahl Null oder 1 bedeuten,
zum optischen Aufhellen von Fasern und Lacken aus Acetylcellulose, Wolle und Polyamid.

9. Mittel zum optischen Aufhellen von Fasern und Lacken aus Acetylcellulose, Wolle und Polyamid, dadurch gekennzeichnet, dass sie eine oder mehrere Pyrazolinverbindungen der Formel

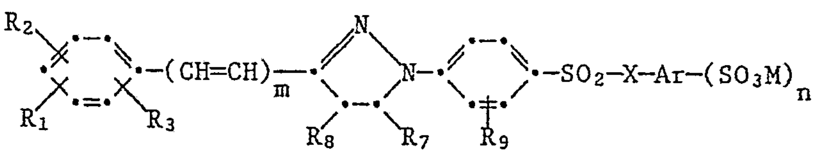

(1)

worin

X = 1,4-Phenylen, $>C=CH_2$, $>CH\text{-}CH_2OH$ oder, falls n = 1 ist oder $R_7$ oder $R_8$ einen $SO_3M$-Rest aufweist, auch eine Methylen-, $-CH_2CH(OH)-CH_2O-$ oder eine unverzweigte $C_{1-4}$-Alkylenoxy-Gruppe, eine direkte Bindung oder Sauerstoff,
$R_1$, $R_2$, $R_3$ = unabhängig voneinander Wasserstoff, Chlor oder $C_{1-4}$-Alkyl,
$R_7$ = $C_{1-4}$-Alkyl, Chlorphenyl, Wasserstoff, Phenyl oder $-C_6H_4-SO_3M$
$R_8$ = H, $C_{1-4}$-Alkyl, $-CH_2SO_3M$, Carboxy, $C_{2-5}$-Carbalkoxy, Carbamoyl oder Carboxymethyl,
$R_9$ = H, Chlor oder $C_{1-4}$-Alkyl
Ar = einen gegebenenfalls nicht chromophor substituierten Phenylrest oder falls X eine direkte Bindung ist auch einen Naphthalinrest,
M = Wasserstoff oder ein Aequivalent eines nichtchromophoren Kations und
m und n die Zahl Null oder 1 bedeuten, enthalten.